(19) Europäisches Patentamt
European Patent Office
Office européen des brevets

(11) Numéro de publication : **0 265 468 B1**

# (12) FASCICULE DE BREVET EUROPEEN

(45) Date de publication du fascicule du brevet : **24.07.91 Bulletin 91/30**

(51) Int. Cl.[5] : **A61K 7/00,** A61K 9/50

(21) Numéro de dépôt : **87902548.4**

(22) Date de dépôt : **17.04.87**

(86) Numéro de dépôt international : **PCT/FR87/00128**

(87) Numéro de publication internationale : **WO 87/06460 05.11.87 Gazette 87/24**

## (54) DISPERSION DE SPHERULES LIPIDIQUES.

(30) Priorité : **22.04.86 FR 8605776**

(43) Date de publication de la demande : **04.05.88 Bulletin 88/18**

(45) Mention de la délivrance du brevet : **24.07.91 Bulletin 91/30**

(84) Etats contractants désignés : **BE IT**

(56) Documents cités :
**EP-A- 0 120 722**
**WO-A-85/04880**
**FR-A- 2 315 991**
**GB-A- 2 026 340**

(73) Titulaire : **L'OREAL**
**14, Rue Royale**
**F-75008 Paris (FR)**

(72) Inventeur : **HANDJANI, Rose-Marie**
**24-26, rue du Cotentin**
**F-75015 Paris (FR)**
Inventeur : **RIBIER, Alain**
**16, rue Caffarelli**
**F-75003 Paris (FR)**

(74) Mandataire : **Peuscet, Jacques et al**
**Cabinet Peuscet 68, rue d'Hauteville**
**F-75010 Paris (FR)**

**EP 0 265 468 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention concerne une composition à usage cosmétique consistant en une dispersion aqueuse de sphérules lipidiques.

On sait que certains lipides possèdent la propriété de former, en présence d'eau, des phases mésomorphes dont l'état d'organisation est intermédiaire entre l'état cristallin et l'état liquide. Parmi les lipides qui donnent naissance à des phases mésomorphes, il a déjà été indiqué que certains peuvent gonfler en solution aqueuse pour former des sphérules dispersées dans le milieu aqueux, ces sphérules étant constituées par des couches multi-moléculaires et de préférence des couches bi-moléculaires.

Dans le brevet français n° 2315991, on a déjà décrit des dispersions de sphérules lipidiques ; ces sphérules sont caractérisées par leur structure en feuillets constituée d'une pluralité de couches lipidiques séparées les unes des autres par des couches de phase aqueuse ; elles peuvent ainsi servir à encapsuler, dans les compartiments aqueux compris entre les couches lipidiques, des substances actives hydrosolubles et à les protéger des conditions extérieures. Les composés lipidiques utilisables pour constituer de telles sphérules peuvent être des composés ioniques, auquel cas on obtient des liposomes, ou des composés non-ioniques, auquel cas on obtient des niosomes.

Dans les brevets français n° 2485921 et 2490504, on a également décrit des compositions consistant en une dispersion aqueuse de sphérules du type précité dans la phase aqueuse externe desquelles on prévoyait une dispersion d'huile. On a constaté que, de façon surprenante, la présence de sphérules lipidiques permettait de stabiliser la dispersion d'huile et qu'en outre, on obtenait, avec de telles compositions, un effet combiné des sphérules et des gouttelettes d'huile.

Dans le brevet français numéro 2543018, on a proposé, en outre, un procédé de préparation de vésicules lipidiques unilamellaires ayant un diamètre moyen supérieur à 1000 Å.

On soulignera ici que les dispersions aqueuses de vésicules lipidiques ont un intérêt tout particulier en cosmétique où elles présentent un avantage considérable par rapport à l'utilisation bien connue des émulsions, car elles permettent précisément d'éviter l'utilisation simultanée d'un émulsionnant et d'une huile, combinaison qui peut être irritante pour la peau. Par ailleurs, elles permettent d'introduire des substances hydrophiles dans un milieu essentiellement lipophile, d'où un effet de protection de ces substances vis-à-vis des différents agents d'altération possibles, comme les oxydants.

Lorsque l'on prépare des liposomes ou des niosomes, divers additifs peuvent être associés aux composés lipidiques ioniques ou non-ioniques, en vue de modifier la perméabilité ou la charge superficielle des sphérules. On a cité, à cet égard, un certain nombre de ces additifs dans les brevets français précités. On sait que l'incorporation de molécules à charges électriques dans les parois des vésicules, liposomes ou niosomes, affecte les propriétés de ces multi-couches. Le rôle des lipides chargés est d'améliorer la stabilité des vésicules en prévenant leur floculation et, de ce fait, leur fusion, même en présence d'électrolytes, et de permettre l'augmentation du taux d'encapsulation de substances hydrosolubles en accroîssant l'épaisseur des feuillets aqueux qui séparent les multi-couches lipidiques.

Pour améliorer les propriétés topiques de ces vésicules lipidiques, on peut songer à incorporer à la phase lipidique qui les constitue, des composés présentant une action bénéfique pour le revêtement cutané, comme des polypeptides ou des composés renfermant des fractions polypeptidiques. Cependant, il est connu que les polypeptides ont, en règle générale, une action déstabilisante sur les vésicules lipidiques, la conséquence gênante étant une augmentation de la perméabilité.

D'une façon surprenante, la Société Déposante a découvert que l'utilisation d'une famille spécifique de composés lipoprotidiques, comme additifs à la phase lipidique des sphérules, conduit à l'amélioration recherchée de l'effet topique sans que l'on observe une augmentation notable et rédhibitoire de la perméabilité, à la condition toutefois de respecter une gamme spécifiée de proportions pour ces lipoprotides.

Parallèlement à ce maintien surprenant de la capacité d'encapsulation des vésicules lipidiques, on conserve l'effet de stabilité de la dispersion.

Les lipoprotides selon l'invention présentent tous, d'une part, une partie lipidique par laquelle ils s'incorporent dans la membrane vésiculaire et, d'autre part, une partie protidique, qui est dirigée vers l'extérieur de ladite membrane et va ainsi pouvoir, lors de l'application sur le revêtement cutané ou les cheveux, agir directement sur ceux-ci.

La présente invention a donc pour objet le produit industriel nouveau que constitue une composition cosmétique ou pharmaceutique consistant en une dispersion, dans un milieu aqueux D, de sphérules lipidiques constituées de couches moléculaires organisées encapsulant une phase aqueuse E, le(s) lipide(s) constitutif(s) desdites couches étant un (ou des) amphiphile(s) ionique(s) ou non-ionique(s), caractérisée par le fait qu'à la phase lipidique propre desdites sphérules, est associé au moins un lipoprotide exempt de fonction sulfhydryle choisi parmi les dérivés mono- ou polyacylés d'amino-acides ou de polypeptides dans lesquels le reste acyle

R-CO comporte une chaîne hydrocarbonée R en $C_{13}$-$C_{19}$, au moins une des fonctions qui relie la chaîne polypeptidique ou le reste d'amino-acide à la chaîne lipophile étant une fonction amide, les fonctions carboxyliques de la chaîne polypeptidique ou du reste d'amino-acide pouvant être, le cas échéant, partiellement ou totalement neutralisées par un ou plusieurs cations alcalins, un ion ammonium ou un ammonium substitué dérivé d'une amine, ledit (ou lesdits) lipoprotide(s) étant présent(s) à un taux de 1 à 15% en poids par rapport au poids total de la phase lipidique propre.

Dans cette définition, dans toute la description et dans les revendications, on appelle "phase lipidique propre", la quantité des lipides, qui constituent les parois des vésicules.

De préférence, le (ou les) reste(s) acyle des lipoprotides utilisés est (ou sont) choisi(s) parmi les restes palmitoyle, myristoyle, stéaroyle, oléoyle, linoléoyle et linolénoyle.

La chaîne protidique des lipoprotides utilisés est notamment dérivée du collagène ou de l'hydroxyproline.

Parmi les lipoprotides particuliers utilisables pour la mise en oeuvre de la présente invention, on peut mentionner le lipoaminoacide palmitoyl collagénique, le dérivé dipalmitoyl-O-N de l'hydroxyproline, le linoléate d'hydroxyproline, le stéaroylglutamate de sodium, le stéaroyl tripeptide de collagène et l'oléoyl tétra et pentapeptide de collagène.

La gamme de proportions spécifiée pour les lipoprolides (1 à 15% en poids par rapport à la phase lipidique propre) résulte d'un meilleur compromis entre l'obtention d'un effet cosmétique sensible des lipoprotides introduits et la conservation de l'imperméabilité des vésicules dans des limites acceptables. Si le taux de lipoprotides était choisi avec une valeur inférieure à 1%, on n'observerait plus l'effet cosmétique. En revanche, si ce taux devait dépasser 15%, la perméabilité des vésicules serait trop forte pour les rendre valablement utilisables.

Pour réaliser la dispersion dans la phase aqueuse D des sphérules lipidiques, on peut utiliser n'importe lequel des procédés antérieurement connus et décrits.

On peut, par exemple, utiliser le procédé, qui consiste à dissoudre les lipides dans un solvant volatil, à former un film mince de lipides sur les parois d'un flacon par évaporation du solvant, à introduire dans ledit flacon la phase aqueuse E à encapsuler et à agiter le mélange mécaniquement jusqu'à l'obtention de la dispersion de sphérules à la taille désirée ; dans ce cas, les phases aqueuses D et E sont nécessairement identiques.

On peut aussi utiliser le procédé décrit dans le brevet français n° 2315991, qui consiste à former une phase lamellaire plane par introduction de la phase aqueuse à encapsuler E dans les lipides liquides, à une température légèrement supérieure à la température de fusion des lipides, à ajouter ensuite à la phase lamellaire obtenue une phase aqueuse de dispersion D, qui peut être identique ou non à la phase aqueuse E, et à agiter énergiquement, par exemple mécaniquement, pour obtenir le passage de la phase lamellaire plane à une dispersion, dans la phase aqueuse D, de sphérules lipidiques encapsulant la phase aqueuse E. Selon les moyens utilisés pour réaliser la dispersion (ultra-disperseur, homogénéiseur et/ou ultrasons) et selon le temps d'agitation (de 15 minutes à quelques heures), on obtient des sphérules, dont le diamètre moyen varie de 0,025 à 5 micromètres (microns) environ.

Le procédé sus-indiqué convient particulièrement bien, lorsque l'on souhaite utiliser des sphérules multilamellaires. Dans le cas où l'on désire des sphérules unilamellaires, on peut utiliser, pour leur préparation, le procédé décrit dans le brevet français numéro 2543018 ; selon ce procédé, on solubilise les lipides destinés à former le feuillet des vésicules dans au moins un solvant insoluble dans l'eau ; on conditionne la solution lipidique à l'état liquide, dans un récipient, à une pression $P_1$ et à une température $\theta_1$ ; on conditionne la phase aqueuse à encapauler E à une pression $P_2$ et à une température $\theta_2$, et on injecte la solution lipidique dans la phase aqueuse de telle sorte que le(s) solvant(s) de la solution lipidique se vaporise(nt) lorsqu'il(s) arrive(nt) au contact de ladite phase aqueuse, ladite injection étant réalisée avec un débit réduit pour constituer initialement des gouttelettes, la pression $P_2$ étant inférieure à la pression $P_1$ et à la tension de vapeur du (ou des) solvant(s) dans lesdites gouttelettes à la température $\theta_2$.

Les lipoprotides selon l'invention peuvent être ajoutés à n'importe quel moment avant la formation des vésicules c'est-à-dire, lorsque l'on passe par la formation d'une phase lamellaire, soit avant la préparation de ladite phase lamellaire, soit après.

Les lipides utilisés pour la préparation des sphérules sont des amphiphiles d'origine naturelle ou synthétique, ioniques ou non-ioniques, comportant, par molécule, une ou plusieurs longue(s) chaîne(s) hydrocarbonée(s), saturée(s) ou insaturée(s), linéaire(s) ou ramifiée(s), ayant notamment de 8 à 30 atomes de carbone, comme les chaînes oléique, lanolique, tétradécylique, hexadécylique, isostéarylique, laurique ou alcoylphényle, et un ou plusieurs groupement(s) hydrophile(s) pris parmi les groupes hydroxyle, étheroxyde, carboxyle, phosphate et amine.

Parmi les amphiphiles ioniques, on préfère utililiser les phospholipides naturels (par exemple la lécithine d'oeuf ou de soja ou la sphingomyéline), les phospholipides de synthèse (par exemple, la dipalmitoyl-phosphatidylcholine ou la lécithine hydrogénée) ; on peut aussi utiliser les composés amphotères comportant deux

chaînes lipophiles ou une association de deux ions organiques à longue chaîne de signes opposés, ainsi que les composés anioniques.

Parmi les composés anioniques, on citera ceux qui sont décrits dans la demande de brevet luxembourgeois n° 85 971 déposée le 23 Juin 1985 et qui sont représentés par la formule :

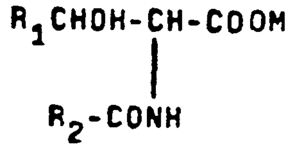

formule dans laquelle :

— $R_1$ désigne un radical alcoyle ou alcényle en $C_7$-$C_{21}$ ;

— $R_2$ désigne un radical hydrocarboné, saturé ou insaturé, en $C_7$-$C_{31}$ ; et

— M représente H, Na, K, $NH_4$ ou un ion ammonium substitué dérivé d'une amine.

Les composés anioniques définis au précédent paragraphe peuvent être obtenus par le procédé de préparation mentionné dans la demande de brevet français 2588256.

Pour les amphiphiles non-ioniques, on préfère que les groupes hydrophiles soient des groupes polyoxyéthylénés ou polyglycérolés, ou des groupes dérivant d'esters de polyols oxyéthylénés ou non ou encore des dérivés d'hydroxyamides. Avantageusement ces composés lipidiques non-ioniques sont choisis dans le groupe formé par

— les éthers de polyglycérol, linéaires ou ramifiés, de formules respectives :

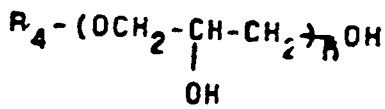

et

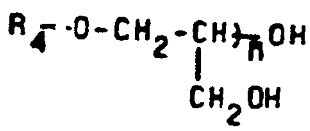

$\bar{n}$ étant une valeur statistique moyenne comprise entre 1 et 6, $R_4$ étant une chaîne aliphatique, linéaire ou ramifiée, saturée ou insaturée, comprenant de 12 à 30 atomes de carbone, les radicaux hydrocarbonés des alcools de lanoline, les restes hydroxy-2 alkyle des α-diols à longue chaîne comprenant de 8 à 30 atomes de carbone;

— les éthers de polyglycérol, linéaires ou ramifiés, comportant deux chaînes grasses ;

— les alcools gras polyoxyéthylénés ;

— les stérols polyxyéthylénés ;

— les éthers de polyols ;

— les esters de polyols oxyéthylénés ou non et, en particulier, les esters de sorbitol polyoxyéthylénés ;

— les glycolipides d'origine naturelle ou synthétique, par exemple les cérébrosides ;

— les hydroxyamides tels que ceux qui sont décrits dans la demande de brevet luxembourgeois n° 85971 déposée le 23 Juin 1985 et qui sont représentés par la formule :

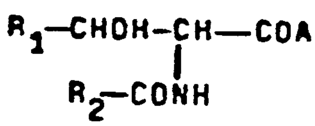

formule dans laquelle :

— $R_1$ désigne un radical alcoyle ou alcényle ayant de 7 à 21 atomes de carbone ;

— $R_2$ désigne un radical hydrocarboné, saturé ou insaturé, ayant de 7 à 31 atomes de carbone ;

— COA désigne un groupement choisi parmi les deux groupements suivants :

— un reste

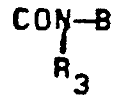

B étant un radical dérivé d'amines primaires ou secondaires, mono- ou polyhydroxylées et $R_3$ désignant un atome d'hydrogène ou un radical méthyle, éthyle ou hydroxyéthyle ;

— COOZ, Z représentant le reste d'un polyol ayant de 3 à 7 atomes de carbone.

De façon connue, divers autres additifs peuvent être associés aux composés lipidiques en vue de modifier la perméabilité ou la charge superficielle des sphérules. On citera, à cet égard, l'addition éventuelle des alcools et diols à longue chaîne, comprenant de 8 à 30 atomes de carbone, des stérols, par exemple le cholestérol et le β-sitostérol, des amines à longue chaîne comprenant de 8 à 30 atomes de carbone, des hydroxyalkylamines, des amines grasses polyoxyéthylénées, des esters d'amino-alcools à longue chaîne comprenant de 8 à 30 atomes de carbone, de leurs sels, des esters phosphoriques d'alcool gras, par exemple le dicétylphosphate de sodium et des alkyl-sulfates, par exemple le cétylsulfate de sodium, des dérivés ioniques des stérols.

Avantageusement, on peut utiliser, pour constituer la dispersion de sphérules, de 0,5 à 25% en poids d'amphiphile(s) par rapport au poids total de la dispersion de sphérules à obtenir.

On peut prévoir que les parois des sphérules renferment au moins une substance liposoluble active telle que, par exemple, un agent kératolytique comme l'acide rétinoïque, ou un agent anti-inflammatoire comme le 17-valérate de β-méthasone, ou encore un anti-oxydant comme la vitamine E et son acétate ou le palmitate d'ascorbyle, ce qui est particulièrement intéressant lorsqu'on envisage des applications topiques.

On peut aussi prévoir que la phase aqueuse E à encapsuler dans les sphérules soit une solution aqueuse de substance active, de préférence isoosmotique par rapport à la phase D de la dispersion. Les phases D et E peuvent être identiques.

La phase aqueuse E encapsulée dans les sphérules ou la phase aqueuse externe D contient, par exemple, au moins une substance cosmétique hydrosoluble prise dans le groupe formé par des humectants, tels que la glycérine, le sorbitol, le pentaérythritol, l'inositol, l'acide pyrrolidone-carboxylique et ses sels ; des agents de brunissage artificiel, tels que la dihydroxyacétone, l'érythrulose, le glycéraldéhyde, les γ-dialdéhydes, tels que l'aldéhyde tartrique, éventuellement associés à d'autres agents de coloration de la peau ; des agents anti-solaires ; des anti-perspirants ; des déodorants ; des astringents ; des produits rafraîchissants, toniques, cicatrisants, kératolytiques, dépilatoires ; des extraits de tissus d'animaux ou végétaux ; des eaux parfumées ; des colorants hydrosolubles ; des agents anti-pelliculaires ; des agents anti-séborrhéiques ; des oxydants, tels que l'eau oxygénée, et des réducteurs, tels que l'acide thioglycolique et ses sels.

Dans le cas d'une composition utilisable en pharmacie, la phase aqueuse E encapsulée dans les sphérules ou la phase aqueuse externe D contient, de préférence, au moins un produit pris dans le groupe formé par les vitamines, les hormones, les enzymes, tels que la superoxyde dismutase, les vaccins, les anti-inflammatoires, tels que l'hydrocortisone, les antibiotiques et les bactéricides.

On peut également prévoir que la phase aqueuse D entourant les sphérules contienne au moins une phase liquide L non miscible à l'eau dispersée dans ladite phase aqueuse D. Cette phase liquide L non miscible à l'eau peut être une huile ou un constituant pris dans le groupe formé par les hydrocarbures, les carbures halogénés, les polysiloxanes, les esters d'acides organiques, les éthers et polyéthers. Avantageusement, la quantité de phase liquide L non miscible à l'eau, dispersée dans la phase aqueuse D, est comprise entre 2 et 70% en poids par rapport au poids total de la composition, la proportion pondérale relative de lipide(s) amphiphile(s) constitutif(s) de sphérules par rapport à la (ou aux) phase(s) liquide(s) non miscible(s) à l'eau, dispersée(s), étant comprise entre 0,02/1 et 10/1.

L'huile utilisée pour être dispersée dans la phase aqueuse D est avantageusement prise dans le groupe formé par les esters d'acides gras et de polyols, notamment les triglycérides liquides, et les esters d'acides gras et d'alcools ramifiés de formule $R_5$-$COOR_6$, formule dans laquelle $R_5$ représente le reste d'un acide gras supérieur comportant de 7 à 19 atomes de carbone et $R_6$ représente une chaîne hydrocarbonée ramifiée contenant de 3 à 20 atomes de carbone. Dans un tel cas, si l'huile est un ester d'acides gras et de polyols, on préfère qu'elle soit choisie dans le groupe formé par les huiles de tournesol, de maïs, de soja, de courge, de pépins de raisins, de jojoba, de sésame et le tri-caprocaprylate de glycérol ; si, au contraire, l'huile est un ester d'acides gras supérieur et d'alcool ramifié, on préfère que ladite huile soit l'huile de Purcellin.

Pour constituer la phase liquide L non miscible à l'eau, on peut encore avantageusement choisir l'hexadécane, l'huile de paraffine, le perhydrosqualène, la perfluorotributylamine, le perfluorodécahydronaphtalène et l'huile de silicone volatile.

On peut également prévoir que la phase aqueuse D, qui entoure les sphérules, contienne au moins un adjuvant pris dans le groupe formé par les opacifiants, les gélifiants, les arômes, les parfums, les filtres anti-solaires et les colorants, ceux de ces adjuvants, qui sont liposolubles, pouvant être dissous dans la phase liquide L non miscible à l'eau dispersée dans la phase aqueuse D, au cas où on utilise une telle dispersion.

Si le liquide non miscible à l'eau dispersé rajouté dans la phase aqueuse continue, qui entoure les sphérules, doit contenir des adjuvants dissous, la dissolution de ces adjuvants est réalisée avant d'effectuer la dispersion.

De tels adjuvants peuvent être, par exemple, des filtres anti-solaires, tels que le paradiméthylamino benzoate de 2-éthyl hexyle ou des substances destinées à améliorer l'état des peaux sèches ou séniles, en particulier des insaponifiables tels que des insaponifiables de soja, d'avocat, des tocophérols, des vitamines E, F, des anti-oxydants.

La dispersion d'huile dans l'eau qui constitue le milieu externe de la dispersion de sphérules peut contenir au moins un additif, notamment un gélifiant ou un parfum. L'additif est ajouté à la dispersion en même temps que l'huile. Le gélifiant peut être introduit à une concentration variant entre 0,1 et 2%, ces pourcentages étant exprimés en poids par rapport au poids total de la composition. Parmi les gélifiants utilisables, on peut citer les dérivés de cellulose, tels que l'hydroxyéthyl cellulose ; des polymères synthétiques ; des dérivés d'algues, tels que le satiagum ou encore des gommes naturelles, telles que l'adragante. On préfère utiliser, à titre de gélifiants, l'hydroxyéthyl cellulose, l'acide polyacrylique réticulé vendu par la société "GOODRICH" sous la dénomination commerciale "CARBOPOL 940", du satiagum ou encore de l'adragante.

Quand on réalise une composition comportant une dispersion de liquide(s) non miscible(s) à l'eau, on constate que cette dispersion est stable sans utilisation d'émulsionnant.

Si la dispersion de sphérules comporte des sphérules de plusieurs types, par exemple des niosomes et des liposomes, on prépare séparément les deux types de sphérules et on mélange les deux dispersions.

Pour mieux illustrer l'objet de la présente invention, on va indiquer maintenant des résultats d'essais démontrant que l'introduction de lipoprotides selon l'invention, dans la phase lipidique de sphérules en dispersion dans l'eau maintient, pour ces sphérules, une perméabilité et un taux d'encapsulation tout-à-fait acceptable, dès l'instant que la borne supérieure de la gamme spécifiée des pourcentages de ces lipoprotides n'est pas dépassée.

Ces essais sont résumés dans le tableau ci-après.

TABLEAU

| Phase lipidique constituée par A, Ch et X, le rapport en poids A/Ch étant de 1/1 | | Gonflement au glucose en µl par mg de phase lipidique | Perméabilité (%) après (n) jours | | |
|---|---|---|---|---|---|
| X | Pourcentage en poids de X par rapport à(A+Ch) | | (n) = 0 | (n) = 8 | (n) = 15 |
| B | 5 | 9,1 | 0 | 3 | 8 |
| | 10 | 9,5 | 0 | 4 | 9 |
| | 15 | 8,5 | 1 | 9 | 14 |
| | 20 (*) | 5,8 | 13 | 20 | 24 |
| C | 5 | 6,2 | 1 | 3 | 6 |
| | 10 | 6,7 | 1 | 25 | 34 |
| | 20(*) | 3,5 | 31 | 42 | 57 |
| D | 10 | 11,8 | 10 | 16 | 18 |
| E | 10 | 14,5 | 5 | 5 | 5 |
| F | 10 | 9,6 | 10 | 13 | 14 |
| G | 10 | 9,8 | 3 | 11 | 17 |

(*) expérience comparative ne faisant pas partie de l'invention

Dans ce tableau, les abréviations A, Ch, B, C, D, E, F et G ont les significations respectives suivantes :

A = lipide non-ionique représenté par la formule suivante :

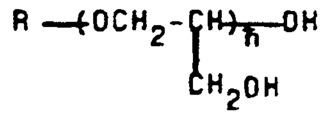

dans laquelle R = $C_{16}H_{33}$ et $\bar{n}$ est une valeur statistique moyenne égale à 3.

Ch = Cholestérol

B = Lipoaminoacide palmitoyl collagénique, représenté par la formule :

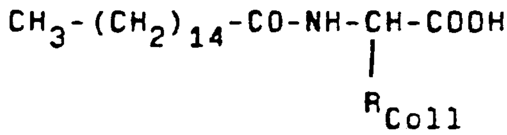

dans laquelle $R_{Coll}$ est le reste polypeptidique du collagène, ce produit étant commercialisé sous la dénomination "PCo" par la Société "RHONE-POULENC".

C = le dérivé dipalmitoyl-O-N de l'hydroxyproline représenté par la formule :

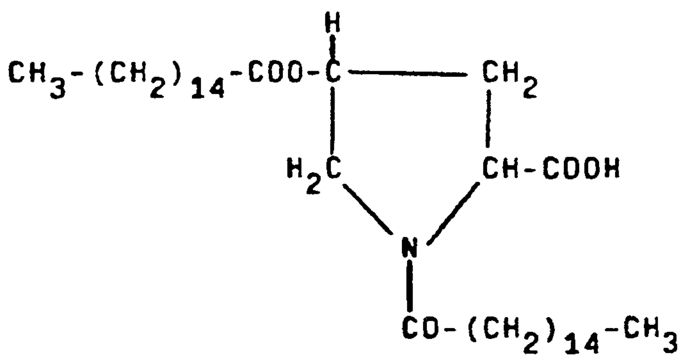

de poids moléculaire 607, les fractions lipidique et protidique représentant respectivement 79 et 21% en poids, cet acide étant commercialisé par la Société "RHONE-POULENC" sous la dénomination "D.P.H.P.".

D = Linoléate d'hydroxyproline commercialisé sous la dénomination "AMINOEFADERMA" par la Société "VEVY".

E = Stéaroyl glutamate de sodium commercialisé par la Société "AJINOMOTO" sous la dénomination "ACYLGLUTAMATE H.S 11"

F = Stéaroyl tripeptide de collagène commercialisé sous la dénomination "LEXEIN A 200" par la société "INOLEX".

G = Oléoyl tétra et pentapeptide de collagène commercialisé sous la dénomination "LAMEPON L PO" par la société "GRÜNAU".

On va donner ci-après quelques exemples de préparation mettant en oeuvre l'invention et quelques exemples de formulation illustrant l'utilisation des dispersions de sphérules selon l'invention.

La préparation des formulations cosmétiques ou pharmaceutiques données dans les exemples ci-dessous s'effectue en 1 ou 2 phases.

Dans une première phase, on fabrique une dispersion aqueuse selon le procédé décrit dans le brevet français 2315991.

La dispersion aqueuse de sphérules lipidiques est préparée à partir :

— d'un lipide amphiphile non ionique ou anionique ou amphotère,
— d'un lipoprotide présentant une ou plusieurs fonctions acides libres ou neutralisées sous forme de sels,
— d'un stérol, facultatif, et de préférence le cholestérol,
— de substances actives facultatives de nature liposoluble et/ou de nature hydrosoluble et d'eau déminéralisée.

Dans une seconde phase, facultative, selon le caractère cosmétique ou pharmaceutique de la formulation, on pourra ajouter une phase liquide non miscible à l'eau dans le milieu externe.

On pourra également ajouter différents additifs cosmétiques tels que parfum et gélifiants par exemple.

EXEMPLE 1 : CREME DE SOINS POUR PEAUX SECHES

1ère phase de préparation :

Dans un bécher en acier inoxydable, on pèse les produits suivants :

- lipide amphiphile non ionique de formule 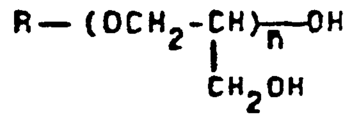 (formule dans laquelle R est un radical hexadécyle et $\bar{n}$ a une valeur statistique moyenne égale à 3) .......................... 3,5 g
- cholestérol .......................... 3,5 g

On réalise le mélange de ces deux produits par fusion à la température de 110°C sous atmosphère d'azote, puis on ramène la température du mélange fondu à 80°C. On ajoute ensuite 1 g du lipoaminoacide palmitoyl collagénique commercialisé sous la référence "PCO" par la société "RHONE-POULENC" de formule

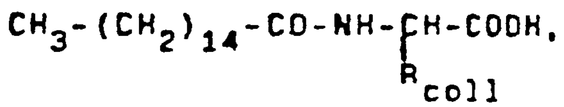

formule dans laquelle $R_{coll}$ est un reste polypeptidique du collagène.

Après homogénéisation du mélange des trois produits à la température de 80°C, on ajoute 3 g de glycérine dissoute dans 20 g d'eau déminéralisée.

On homogénéise le mélande obtenu à la température de 80°C.

On ajoute alors les produits suivants :

- parahydroxybenzoate de méthyle (stabilisateur) .................... 0,3 g
- eau déminéralisée .................... 22,5 g

A la température de 80°C, on homogénéise le mélange à l'aide d'un ultradisperseur "Virtis" jusqu'à ce que la taille moyenne des vésicules obtenues soit de 0,5 micromètre (micron).

2ème phase de préparation :

On ajoute au mélange obtenu 25 g d'huile de sésame. On soumet le tout à l'action d'un ultradisperseur "Virtis" jusqu'à ce que les globules d'huile aient un diamètre moyen d'environ 1 micromètre (micron).

On ajoute enfin les produits suivants :

- Parfum............................ 0,4 g
- Acide polyacrylique réticulé vendu par la société "GOODRICH" sous la dénomination commerciale "CARBOPOL 940".......... 0,4 g
- Triéthanolamine.................... 0,4 g
- Eau déminéralisée.................. 20,0 g

Cette crème, appliquée en utilisation topique une fois par jour sur des sujets à peau sèche, donne des résultats satisfaisants après 20 jours d'application.

EXEMPLE 2 : BASE DE SOINS POUR LES ONGLES

Dans un bécher en acier inoxydable, on pèse les produits suivants :

- lipide amphiphile non ionique de formule $R - (OCH_2-\underset{\displaystyle CH_2OH}{\underset{|}{CH}})_{\bar{n}}-OH$ (formule dans laquelle R est un radical hexadécyle et $\bar{n}$ a une valeur statistique moyenne égale à 3)...................... 8,5 g
- cholestérol....................... 8,5 g

On réalise le mélange de ces deux produits par fusion à la température de 110°C sous atmosphère d'azote, puis on ramène la température du mélange fondu à 70°C et on ajoute 3 g de stéaroylglutamate de sodium vendu par la société "AJINOMOTO" sous la dénomination "ACYLGLUTAMATE HS11".

Après homogénéisation du mélange des trois produits à la température de 70°C, on ajoute 5 g de glycérine dissoute dans 50 g d'eau déminéralisée. On homogénéise le mélange obtenu à la température de 70°C. On ajoute alors les produits suivants :

- parahydroxybenzoate de méthyle (stabilisateur).................... 0,3 g
- eau déminéralisée.................. 24,3 g
- parfum............................ 0,4 g

A la température de 70°C, on homogénéise le mélange à l'aide d'un ultradisperseur "Virtis" jusqu'à ce que la taille moyenne des vésicules obtenues soit d'environ 0,3 micromètre (micron).

Après application deux fois par jour de la base de soins pour les ongles, au bout de quelques jours, on observe un lissage de la surface des ongles ainsi qu'un durcissement.

EXEMPLE 3 : CONCENTRE POUR LE TRAITEMENT DES PEAUX IRRITEES

Dans un ballon rond de 1 litre, on dissout, dans 200 ml d'un mélange solvant (chloroforme/méthanol dans le rapport 2/1), les produits suivants :

| | | |
|---|---|---|
| - lécithine de soja vendue sous la dénomination commerciale "EPIKURON E 200" par la société "LUKAS MEYER" | 12,0 | g |
| - cholestérol | 4,0 | g |
| - DL-α-tocophérol | 1,0 | g |
| - linoléate d'hydroxyproline (produit commercialisé sous le nom "AMINOEFADERMA" par la société "VEVY" | 1,5 | g |

On évapore le solvant à l'aide d'un évaporateur rotatif et on élimine les dernières traces de solvant par passage à la pompe à palette pendant une heure. On met en contact l'association de lipides obtenue avec 40 g d'eau déminéralisée mélangée à 3 g de glycérine. On homogénéise le mélange à la température de 40°C.

On ajoute ensuite les produits suivants :

| | | |
|---|---|---|
| - parahydroxybenzoate de méthyle (stabilisateur) | 0,3 | g |
| - eau déminéralisée | 37,5 | g |
| - parfum | 0,7 | g |

On soumet le tout à l'action d'un ultradisperseur du type "Virtis" jusqu'à ce que la taille moyenne des vésicules obtenues soit inférieure au micromètre (micron).

La dispersion obtenue, fluide, peut être appliquée sur la peau par projection à partir d'un flacon pompe.

Cette crème, utilisée en application topique deux fois par jour sur des sujets ayant une peau acnéique irritée, diminue l'irritation après une ou deux semaines d'application.

## EXEMPLE 4 : LIPOSERUM POUR LE RAFFERMISSEMENT DE LA PEAU

Dans un bécher en acier inoxydable, on pèse les produits suivants :

| | | |
|---|---|---|
| - lipide amphiphile non-ionique de formule $R-(OCH_2-\underset{CH_2OH}{\underset{\vert}{CH}})_{\bar{n}}-OH$ (formule dans laquelle R est un radical hexadécyle et $\bar{n}$ a une valeur statistique moyenne égale à 3) | 5,4 | g |
| - cholestérol | 5,4 | g |

On réalise le mélange de ces deux produits par fusion à la température de 110°C sous atmosphère d'azote, puis on ramène la température du mélange fondu à 75°C et on ajoute 1,2 g d'un stéaroyl-tripeptide de collagène commercialisé par la société "INOLEX" sous la dénomination commerciale "LEXEIN A 200". On homogénéise le mélange à la température de 75°C.

On ajoute ensuite une partie de la phase aqueuse composée de :

| | | |
|---|---|---|
| - glycérine...................... | 3,0 | g |
| - eau déminéralisée.............. | 17,0 | g |
| - solution aqueuse obtenue par broyage de tissus placentaires animaux, commercialisée par la société "GATTEFOSSE" sous la dénomination commerciale "PHYLDERM"..................... | 20,0 | g |

On homogénéise le mélange obtenu à la température de 70°C.

On ramène la température à 60°C et on ajoute 20 g d'une solution aqueuse à 1% de mannuronate de monométhyltrisilanol vendu par la société "EXYMOL" sous la dénomination commerciale "ALGISIUM". A la température de 60°C, on homogénéise le mélange à l'aide d'un ultradisperseur "Virtis" jusqu'à ce que la taille moyenne des vésicules obtenues soit d'environ 0,5 micromètre (micron). A ce stade de la fabrication, on refroidit à la température ambiante et on amène la dispersion à pH 5,5 par addition d'une solution aqueuse de soude 0,1 N.

On ajoute alors 0,15 g d'un stabilisateur vendu par la société "ROHM et HAAS" sous la dénomination commerciale "KATHON CG" dissous dans 1 g d'eau déminéralisée. On ajoute ensuite 10 g d'une solution aqueuse à 5% de sérum-albumine bovine commercialisée par la société "SILAB". On homogénéise le mélange obtenu et on ajoute 6 g d'huile de silicone volatile. On soumet le tout à l'action d'un ultradisperseur jusqu'à ce que les globules d'huile aient un diamètre moyen inférieur au micromètre (micron).

On ajoute enfin les produits suivants :

| | | |
|---|---|---|
| - polyglucose à chaîne linéaire vendu par la société "ALBAN MULLER" sous la dénomination commerciale "AMIGEL POUDRE"..... | 0,1 | g |
| - eau déminéralisée........q.s.p.. | 100 | g |

Après application deux fois par jour pendant 3 semaines, on note un raffermissement de la peau.

EXEMPLE 5 : LAIT POUR LE SOIN DES PEAUX SECHES

1ère phase de préparation :

Dans un bécher en acier inoxydable, on pèse les produits suivants :

a) lipide amphiphile non ionique de formule :

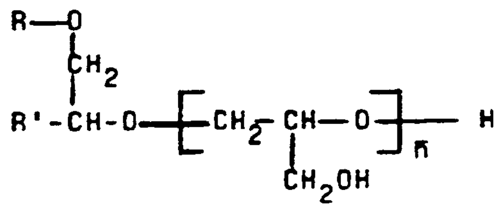

dans laquelle :

- R est un radical dodécyle ;
- R' est un mélange équimolaire des radicaux tétradécyle et hexadécyle ; et
- $\bar{n}$ a une valeur statistique moyenne déterminée par résonance magnétique nucléaire égale à 5,5.................... 7,0 g

b) lipoamino-acide palmitoyl collagénique commercialisé sous la référence "PCO" par la Société "RHONE POULENC" de formule $CH_3-(CH_2)_{14}-CO-NH-CH-COOH$............... 1 g
R

dans laquelle R est un acide aminé obtenu par hydrolyse du collagène.

Après homogénéisation à 45°C, on ajoute 3 g de glycérine dissoute dans 20 g d'eau déminéralisée. On homogénéise le mélange obtenu à 90°C ; on ajoute alors 0,3 g de parahydroxybenzoate de méthyle (stabilisateur) dissous dans 37,4 g d'eau déminéralisée.

A 40°C, on homogénéise le mélange à l'aide d'un ultradisperseur "Virtis" jusqu'à ce que la taille moyenne des sphérules obtenues soit de 0,2 micromètre (micron).

On ajoute alors sous agitation 1,3 g de solution aqueuse de soude normale.

2ème phase de préparation :

On ajoute 15,0 g d'huile de sésame. On soumet le tout à l'action de l'ultradisperseur "Virtis" de façon que la phase externe de la dispersion d'huile présente des globules d'huile dont le diamètre moyen est d'environ 1 micromètre (micron).

On ajoute enfin les substances suivantes :

- Parfum.............................................. 0,4 g
- Acide polyacrylique réticulé vendu sous la dénomination commerciale "CARBOPOL 940" par la société GOODRICH........................ 0,4 g
- Tri-éthanolamine..................................... 0,4 g
- Eau déminéralisée.................................... 13,8 g

Ce lait, appliqué en utilisation topique une fois par jour sur des sujets à peau sèche, donne des résultats satisfaisants après deux semaines d'application.

EXEMPLE 6 — CREME DE SOINS POUR PEAUX ACNEIQUES.

Toute la préparation de cette crème a été effectuée à la lumière jaune d'une lampe à vapeur de sodium.

1ère phase de préparation :

Dans un ballon rond de 1 litre, on dissout, dans 200 ml d'un mélange solvant (chloroforme/méthanol dans le rapport 1/1), les produits suivants :

- lipide non-ionique de formule :

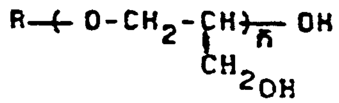

dans laquelle R est un radical hexadécyle et $\bar{n}$ a une valeur statistique moyenne égale à 3 ................ 3,8 g
- cholestérol ............................................ 3,8 g
- acyl-glutamate $HS_{11}$ commercialisé par la Société AJINOMOTO de formule :

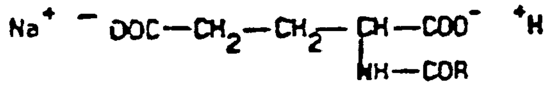

où R est un radical stéaryle ............................ 0,4 g
- acide rétinoïque vendu par la Société "ROCHE" sous la dénomination commerciale "TRETINOINE" ................. 0,025g

On évapore le solvant à l'aide d'un évaporateur rotatif et on élimine les dernières traces de solvant par passage à la pompe à palettes pendant 1 heure.

On met en contact l'association de lipides obtenue avec 20,0 g d'eau déminéralisée mélangée à 3,0 g de glycérine. On homogénéise le mélange obtenu à 80°C. On ajoute alors 0,3 g de parahydroxybenzoate de méthyle (stabilisateur) dissous dans 38,675 g d'eau déminéralisée.

A 60°C, on homogénéise le mélange à l'aide d'un ultradisperseur "Virtis" jusqu'à ce que la taille moyenne des sphérules obtenues soit d'environ 0,3 micromètre (micron).

2ème phase de préparation :

On ajoute 15 g de tricaprocaprylate de glycérol. On soumet le tout à l'action de l'ultradisperseur "Virtis" de façon que la phase externe de la dispersion d'huile présente des globules d'huile, dont le diamètre moyen est d'environ 1 micromètre (micron).

On ajoute enfin les substances suivantes :

- Parfum .......................................... 0,4 g
- Acide polyacrylique réticulé vendu sous la dénomination commerciale "CARBOPOL 940" par la société GOODRICH .......................... 0,4 g
- Tri-éthanolamine .......................................... 0,4 g
- Eau déminéralisée .......................................... 13,8 g

Cette crème, utilisée en application topique deux fois par jour sur des sujets ayant une peau acnéique, permet d'obtenir une amélioration sensible après deux semaines d'application.

EXEMPLE 7 — DISPERSION AQUEUSE DE SPHERULES POUR LE SOIN DU VISAGE.

Dans un bécher en acier inoxydable, on pèse les produits suivants :

- Lipide amphiphile non-ionique utilisé dans l'exemple 5 .......................... 5,6 g
- Cholestérol .......................................... 1,6 g
- Lipoaminoacide palmitoyl·collagénique commercialisé sous la référence "PCO" par la société "RHONE POULENC" de formule $CH_3-(CH_2)_{14}-CO-NH-\underset{R}{CH}-COOH$ dans laquelle R est un acide aminé obtenu par hydrolyse du collagène .......................... 0,8 g

Après homogénéisation à 95°C, on ajoute 5,0 g de glycérine dissoute dans 20,0 g d'eau déminéralisée. On homogénéise le mélange obtenu à 95°C.

On ajoute alors 0,3 g de parahydroxybenzoate de méthyle (stabilisateur) dissous dans 50,7 g d'eau déminéralisée.

A 40°C, on homogénéise le mélange à l'aide d'un ultradisperseur "Virtis" jusqu'à ce que la taille moyenne des sphérules obtenues soit de 0,2 micromètre (micron). On ajoute alors sous agitation 1,0 g d'une solution aqueuse de soude normale.

On ajoute enfin les substances suivantes :

- Parfum .......................................... 0,2 g
- Acide polyacrylique réticulé vendu sous la dénomination commerciale "CARBOPOL 940" par la société GOODRICH .......................... 0,2 g
- Tri-éthanolamine .......................................... 0,2 g
- Eau déminéralisée .......................................... 14,4 g

Cette dispersion, utilisée en application topique pour le soin du visage une fois par jour, donne un résultat très satisfaisant après deux semaines d'application.

EXEMPLE 8 — PREPARATION VESICULAIRE DE CORTICOIDES

Dans un bécher en acier inoxydable, on pèse les produits suivants :

- Lipide amphiphile non-ionique utilisé dans l'exemple 5 .................................... 7,6 g
- Lipoaminoacide de palmitoyl collagénique de formule $CH_3-(CH_2)_{14}-CO-NH-CH(R)-COOH$ dans laquelle R est un acide aminé obtenu par hydrolyse du collagène (commercialisé sous la dénomination "PCO" par la société RHONE POULENC) ........................ 0,4 g
- 17-valérate de β-méthasone (produit commercialisé par la société LARKS) ........................ 0,08 g

On réalise le mélange de ces trois produits par fusion à 90°C. On ajoute 20 g d'eau déminéralisée. On homogénéise le mélange obtenu à 90°C.

On ajoute alors les produits suivants :

- Parahydroxybenzoate de méthyle (stabilisateur) .. 0,3 g
- Glycérine ........................................ 5,0 g
- Eau déminéralisée ................................ 52,02 g

A 40°C, homogénéise le mélange à l'aide d'un ultradisperseur du type "Virtis" jusqu'à ce que la taille moyenne des vésicules obtenues soit de 0,2 micromètre (micron).

On ajoute alors, sous agitation, 0,5 g de solution aqueuse de soude normale.

On ajoute enfin les produits suivants :

- Acide polyacrylique réticulé vendu sous la dénomination commerciale "CARBOPOL 940" par la société GOODRICH ................................ 0,4 g
- Triéthanolamine .................................. 0,4 g
- Eau déminéralisée ................................ 13,3 g

Cette préparation, utilisée en application topique deux fois par jour sur des sujets présentant une dermatose, permet de constater une amélioration sensible après quelques jours d'application.

EXEMPLE 9 — DISPERSION AQUEUSE DE VESICULES LIPIDIQUES

Dans un ballon rond de 1 litre, on dissout, dans 200 ml d'un mélange solvant (chloroforme/méthanol dans le rapport 1/1), les produits suivants :

- Lipide amphiphile non-ionique utilisé dans l'exemple 5.............................. 7,6 g
- Lipoaminoacide palmitoyl collagénique de formule $CH_3-(CH_2)_{14}-CO-NH-\underset{R}{CH}-COOH$

  dans laquelle R est un acide aminé obtenu par hydrolyse du collagène (commercialisé sous la dénomination "PCO" par la société RHONE POULENC)........................... 0,4 g
- Acétate d'α tocophérol (produit commercialisé par la société ROCHE).......................... 0,2 g
- α tocophérol (produit commercialisé par la société ROCHE).............................. 0,2 g
- Palmitate d'ascorbyle (produit commercialisé par la société ROCHE).......................... 0,4 g

On évapore le solvant à l'aide d'un évaporateur rotatif et on élimine les dernières traces de solvant par passage à la pompe à palettes pendant 1 heure. On met en contact l'association de lipides obtenue avec 20 g d'eau déminéralisée. On homogénéise le mélange obtenu à 90°C.

On ajoute alors les produits suivants :

- Parahydroxybenzoate de méthyle (stabilisateur).. 0,3 g
- Glycérine...................................... 5,0 g
- Eau déminéralisée............................. 50,8 g

A 40°C, on homogénéise le mélange à l'aide d'un ultradisperseur "Virtis" jusqu'à ce que la taille moyenne des vésicules obtenues soit de 0,2 micromètre (micron).

On ajoute alors, sous agitation, 0,5 g de solution aqueuse de soude normale.

On ajoute enfin les produits suivants :

- Acide polyacrylique réticulé vendu sous la dénomination "CARBOPOL 940" par la société GOODRICH...................................... 0,4 g
- Triéthanolamine................................ 0,4 g
- Eau déminéralisée............................. 13,8 g

Cette dispersion, utilisée en application topique une fois par jour sur des sujets ayant une peau présentant certains signes de vieillissement, donne des résultats satisfaisants après quatre semaines d'application.

**Revendications**

1. Composition cosmétique ou pharmaceutique consistant en une dispersion dans un milieu aqueux D, de sphérules lipidiques constituées de couches moléculaires organisées encapsulant une phase aqueuse E, le(s) lipide(s) constitutif(s) desdites couches étant des amphiphiles ioniques ou non-ioniques, caractérisée par le

fait qu'à la phase lipidique propre desdites sphérules, est associé au moins un lipoprotide exempt de fonction sulfhydryle choisi parmi les dérivés mono- ou polyacylés d'amino-acides ou de polypeptides, dans lesquels le reste acyle R-CO comporte une chaîne hydrocarbonée R ayant de 13 à 19 atomes de carbone, au moins une des fonctions qui relie la chaîne polypeptidique ou le reste d'amino-acide à la chaîne lipophile étant une fonction amide, les fonctions carboxyliques de la chaîne polypeptidique ou du reste d'amino-acide pouvant être partiellement ou totalement neutralisées par un ou plusieurs cations alcalins, un ion ammonium ou un ammonium substitué dérivé d'une amine, ledit (ou lesdits) lipoprotide(s) étant présent(s) à un taux de 1 à 15% en poids par rapport au poids total de ladite phase lipidique propre.

2. Composition selon la revendication 1, caractérisée par le fait que le (ou les) reste(s) acyle des lipoprotides utilisés est (ou sont) choisi(s) parmi les restes myristoyle, palmitoyle, stéaroyle, oléoyle, linoléoyle et linolénoyle.

3. Composition selon l'une des revendications 1 et 2, caractérisée par le fait que la chaîne protidique des lipoprotides utilisés est dérivée du collagène ou de l'hydroxyproline.

4. Composition selon la revendication 1, caractérisée par le fait que le (ou les) lipoprotide(s) utilisé(s) est (ou sont) choisi(s) dans le groupe formé par le lipoaminoacide palmitoyl collagénique, le dérivé dipalmitoyl—O-N de l'hydroxyproline, le linoléate d'hydroxyproline, le stéaroylglutamate de sodium, le stéaroyltripeptide de collagène et l'oléoyl-(tétra ou penta) peptide de collagène.

5. Composition selon l'une des revendications 1 à 4, caractérisée par le fait que les lipides destinés à constituer les feuillets des sphérules sont des amphiphiles d'origine naturelle ou synthétique, ioniques ou non-ioniques, comportant, par molécule, un ou plusieurs groupement(s) hydrophile(s) pris parmi les groupes hydroxyle, étheroxyde, carboxyle, phosphate et amine.

6. Composition selon la revendication 5, caractérisée par le fait que les amphiphiles ioniques sont pris dans le groupe formé par les phospholipides naturels, tels que la lécithine d'oeuf ou de soja et la sphingomyéline, les phospholipides de synthèse, tels que la dipalmitoyl-phosphatidylcholine ou la lécithine hydrogénée, les composés amphotères et les composés anioniques.

7. Composition selon la revendication 5, caractérisée par le fait que les amphiphiles non-ioniques sont pris dans le groupe formé par :

— les éthers de polyglycérol linéaires ou ramifiés, de formules respectives :

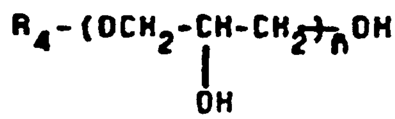

et

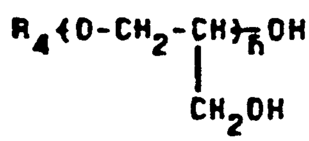

$\bar{n}$ étant une valeur statistique moyenne comprise entre 1 et 6, $R_4$ représentant une chaîne aliphatique, linéaire ou ramifiée, saturée ou insaturée, contenant 12 à 30 atomes de carbone, des radicaux hydrocarbonés des alcools de lanoline ou les restes hydroxy-2 alkyle des α-diols à longue chaîne comprenant de 8 à 30 atomes de carbone ;

— les éthers de polyglycérol, linéaires ou ramifiés, comportant deux chaînes grasses ;
— les alcools gras polyoxyéthylénés ou les stérols polyoxyéthylénés ;
— les éthers de polyols ;
— les esters de polyols, oxyéthylénés ou non ;
— les glycolipides d'origine naturelle ou synthétique ;
— les hydroxyamides représentés par la formule :

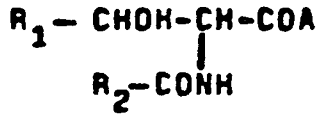

formule dans laquelle :

— $R_1$ désigne un radical alcoyle ou alcényle ayant de 7 à 21 atomes de carbone ;

— $R_2$ désigne un radical hydrocarboné, saturé ou insaturé, ayant de 7 à 31 atomes de carbone ;

— COA désigne un groupement choisi parmi les deux groupements suivants :

— un reste

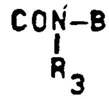

B étant un radical dérivé d'amines primaires ou secondaires, mono- ou polyhydroxylées et $R_3$ désignant un atome d'hydrogène ou un radical méthyle, éthyle ou hydroxyéthyle ;

— COOZ, Z représentant le reste d'un polyol ayant de 3 à 7 atomes de carbone.

8. Composition selon l'une des revendications 1 à 7, caractérisée par le fait que les amphiphiles destinés à former les sphérules, sont associés à des additifs pris dans le groupe formé par des alcools et diols à longue chaîne comprenant de 8 à 30 atomes de carbone, des stérols, des amines à longue chaîne comprenant de 8 à 30 atomes de carbone, des hydroxyalkylamines, des amines grasses polyoxyéthylénées, des esters d'amino alcools à longue chaîne comprenant de 8 à 30 atomes de carbone, et leurs sels, des esters phosphoriques d'alcool gras, des alkyl-sulfates, et des dérivés ioniques de stérols.

9. Composition selon l'une des revendications 1 à 8, caractérisée par le fait qu'elle contient de 0,5 à 25% en poids d'amphiphile(s) constituant les parois de sphérules, ces pourcentages étant exprimés en poids par rapport au poids total de la composition.

10. Composition selon l'une des revendications 1 à 9, caractérisée par le fait que les parois de ces sphérules renferment au moins une substance liposoluble tels que, par exemple, les agents kératolytiques, les agents anti-inflammatoires et les agents anti-oxydants.

11. Composition selon l'une des revendications 1 à 10, caractérisée par le fait que la phase aqueuse E encapsulée dans les sphérules est une solution aqueuse de substance(s) active(s), de préférence isoosmotique par rapport à la phase D qui entoure les sphérules.

12. Composition selon la revendication 11, caractérisée par le fait que les phases aqueuses D et E sont identiques.

13. Composition selon l'une des revendications 11 ou 12, caractérisée par le fait que la phase aqueuse E ou la phase aqueuse externe D renferme au moins une substance cosmétique hydrosoluble prise dans le groupe formé par les humectants, les agents de brunissage artificiel, les agents de coloration de la peau, les agents anti-solaires, les filtres solaires, les agents anti-perspirants, les déodorants, les astringents, les produits rafraîchissants, les produits toniques, les produits cicatrisants, les produits kératolytiques, les produits dépilatoires, les eaux parfumées, les colorants hydrosolubles, les agents anti-pelliculaires, les agents anti-séborrhéiques, les oxydants, les réducteurs et les extraits de tissus animaux ou végétaux.

14. Composition selon l'une des revendications 11 ou 12, caractérisée par le fait que la phase aqueuse E ou la phase aqueuse externe D renferme au moins un produit pris dans le groupe formé par les vitamines, les hormones, les enzymes, les vaccins, les anti-inflammatoires, les antibiotiques et les bactéricides.

15. Composition selon l'une des revendications 1 à 14, caractérisée par le fait qu'au moins une phase liquide L, non miscible à l'eau, est dispersée dans la phase aqueuse D.

16. Composition selon la revendication 15, caractérisée par le fait qu'elle contient de 2 à 70% en poids de phase liquide L non miscible à l'eau par rapport au poids total de la composition, la proportion pondérale relative de lipide(s) amphiphile(s) constitufif(s) de sphérules par rapport à la phase liquide non miscible à l'eau dispersée étant comprise entre 0,02/1 et 10/1.

17. Composition selon l'une des revendications 15 ou 16, caractérisée par le fait que la phase liquide L non miscible à l'eau dispersée dans la phase aqueuse D est choisie dans le groupe constitué par les huiles telles que les esters d'acides gras et de polyols, et les esters d'acides gras et d'alcools ramifiés de formule $R_6$-$COOR_6$, formule dans laquelle $R_5$ représente le reste d'un acide gras supérieur comportant de 7 à 19 atomes de carbone et $R_6$ représente une chaîne hydrocarbonée ramifiée contenant de 3 à 20 atomes de carbone, les hydrocarbures tels que l'hexadécane, l'huile de paraffine, le perhydrosqualène ; les carbures halogénés tels que le perfluorodécahydronaphtalène ; la perfluorotributylamine ; les polysiloxanes ; les esters d'acides organiques, les éthers et polyéthers.

18. Composition selon l'une des revendications 1 à 17, caractérisée par le fait que la phase aqueuse D renferme au moins un adjuvant pris dans le groupe formé par les opacifiants, les gélifiants, les arômes, les parfums, les filtres anti-solaires et les colorants.

19. Composition selon l'une des revendications 15 à 18, caractérisée par le fait que la phase L renferme au moins un parfum et/ou une ou plusieurs substances actives liposolubles.

20. Composition selon la revendication 19, caractérisée par le fait que la substance liposoluble est constituée par un filtre anti-solaire, une substance destinée à améliorer l'état des peaux sèches ou séniles choisie parmi les insaponifiables de soja, d'avocat, les tocophérols, et les vitamines E, F, ou un anti-oxydant.

## Patentansprüche

1. Kosmetisches oder pharmazeutisches Mittel, bestehend aus einer Dispersion in einem wäßrigen Milieu D von Lipidkügelchen, bestehend aus geordneten molekularen Schichten, welche eine wäßrige Phase E einkapseln, wobei das (die) die Schichten bildende(n) Lipid(e) ein ionisches oder nicht-ionisches Amphiphil ist (sind), **dadurch gekennzeichnet**, daß die eigentliche Lipidphase dieser Kügelchen mit mindestens einem Lipoproteid, frei von Sulfhydrylfunktionen assoziiert ist, ausgewählt unter Mono- oder Polyacylderivaten von Aminosäuren oder Polypeptiden, worin der Acylrest R-CO eine Kohlenwasserstoffkette R mit 13 bis 19 Kohlenstoffatomen umfaßt, wobei mindestens eine der Funktionen, welche die Polypeptidkette oder den Aminosäurerest mit der lipophilen Kette verbindet, eine Amidfunktion ist, wobei die Carboxylfunktionen der Polypeptidkette oder des Aminosäurerests teilweise oder vollständig neutralisiert sein können durch ein oder mehrere Alkalikationen, durch ein Ammoniumion oder ein substituiertes Ammoniumderivat eines Amins, und wobei das (oder die) Lipoproteid(e) in einem Anteil von 1-15 Gew.-%, bezogen auf das Gesamtgewicht der eigentlichen Lipidphase, vorliegt (vorliegen).

2. Mittel nach Anspruch 1, dadurch gekennzeichnet, daß der (oder die) Acylrest(e) der verwendeten Lipoproteide ausgewählt ist (sind) unter Myristoyl-, Palmitoyl-, Stearoyl-, Oleoyl-, Linoleoyl- und Linolenoylresten.

3. Mittel nach einem der Ansprüche 1 oder 2, dadurch gekennzeichnet, daß die Proteidkette des verwendeten Lipoproteids von Kollagen oder von Hydroxyprolin abgeleitet ist.

4. Mittel nach Anspruch 1, dadurch gekennzeichnet, daß das (die) verwendete(n) Lipoproteid(e) ausgewählt ist (sind) unter der Palmitoyl-Kollagen-Lipoaminosäure, dem O-N-Dipalmitoylderivat von Hydroxyprolin, dem Hydroxyprolinlinoleat, dem Natriumstearoylglutamat, dem Stearoyltripeptid von Kollagen und dem Oleoyl-(tetra oder penta)-peptid von Kollagen.

5. Mittel nach einem der Ansprüche 1 bis 4, dadurch gekennzeichnet, daß die Lipide zur Ausbildung der Kugelschichten ionische oder nicht-ionische Amphiphile natürlichen oder synthetischen Ursprungs sind, die je Molekül eine oder mehrere hydrophile Gruppen, ausgewählt unter Hydroxyl-, Etheroxid-, Carboxyl-, Phosphat- und Amingruppen, umfassen.

6. Mittel nach Anspruch 5, dadurch gekennzeichnet, daß die ionischen Amphiphile ausgewählt sind unter natürlichen Phospholipiden, wie Ei- oder Sojalecithin und Sphingomyelin, synthetischen Phospholipiden, wie Dipalmitoyl-phosphatidylcholin oder hydriertem Lecithin, amphoteren Verbindungen und anionischen Verbindungen.

7. Mittel nach Anspruch 5, dadurch gekennzeichnet, daß die nicht-ionischen Amphiphile ausgewählt sind unter :

— geradkettigen oder verzweigten Polyglycerinethern der allgemeinen Formeln :

$$R_4-(OCH_2-\underset{\displaystyle OH}{CH}-CH_2)_n-OH$$

und

$$R_4-(O-CH_2-\underset{\displaystyle CH_2OH}{CH})_n-OH$$

worin n einen mittleren statistischen Wert zwischen 1 und 6 bedeutet, $R_4$ eine geradkettige oder verzweigte, gesättigte oder ungesättigte aliphatische Kette mit 12 bis 30 Kohlenstoffatomen bedeutet oder für einen Kohlenwasserstoffrest von Lanolinalkoholen oder für einen 2-Hydroxyalkylrest von langkettigen α-Diolen mit 8 bis 30 Kohlenstoffatomen steht ;

— geradkettigen oder verzweigten Polyglycerinethern mit zwei Fettketten ;

— polyoxyethylenierten Fettalkoholen oder polyoxyethylenierten Sterolen ;

— Polyolethern ;
— gegebenenfalls oxyethylenierten Polyolestern ;
— Glykolipiden natürlichen oder synthetischen Ursprungs ;
— Hydroxyamiden der allgemeinen Formel :

```
R1-CHOH-CH-COA
        |
   R2-CONH
```

worin
$R_1$ einen Alkyl- oder Alkenylrest mit 7 bis 21 Kohlenstoffatomen bedeutet ;
$R_2$ einen gesättigten oder ungesättigten Kohlenwasserstoffrest mit 7 bis 31 Kohlenstoffatomen bedeutet ;
COA für eine Gruppe steht, die unter einer der beiden folgenden Gruppen ausgewählt ist :
— einem Rest

```
CON-B
  |
  R3
```

worin B einen von mono- oder polyhydroxylierten, primären oder sekundären Aminen abgeleiteten Rest bedeutet und $R_3$ ein Wasserstoffatom oder einen Methyl-, Ethyl- oder Hydroxyethylrest bedeutet ;
— COOZ,
worin Z für einen Polyolrest mit 3 bis 7 Kohlenstoffatomen steht.

8. Mittel nach einem der Ansprüche 1 bis 7, dadurch gekennzeichnet, daß die Amphiphilen für die Bildung der Kügelchen assoziiert sind mit Additiven,ausgewählt unter langkettigen Alkoholen und Diolen mit 8 bis 30 Kohlenstoffatomen, Sterolen, langkettigen Aminen mit 8 bis 30 Kohlenstoffatomen, Hydroxyalkylaminen, polyoxyethylenierten Fettaminen, Estern von langkettigen Aminoalkoholen mit 8 bis 30 Kohlenstoffatomen, und den Salzen davon, Phosphorsäureestern von Fettalkoholen, Alkylsulfaten und ionischen Sterolderivaten.

9. Mittel nach einem der Ansprüche 1 bis 8, dadurch gekennzeichnet, daß es 0,5 bis 25 Gew.-% des (der) die Wände der Kügelchen bildenden Amphiphils (Amphiphile) enthält, wobei der Prozentsatz ausgedrückt ist in Prozenten, bezogen auf das Gesamtgewicht des Mittels.

10. Mittel nach einem der Ansprüche 1 bis 9, dadurch gekennzeichnet, daß die Wände der Kügelchen mindestens eine fettlösliche Substanz, wie z.B. keratolytische Verbindungen, antiinflammatorische Verbindungen und Antioxidantien, einschließen.

11. Mittel nach einem der Ansprüche 1 bis 10, dadurch gekennzeichnet, daß die in den Kügelchen eingekapselte, wäßrige Phase E eine, vorzugsweise bezüglich der Phase D, welche die Kügelchen umgibt, isoosmotische wäßrige Lösung einer oder mehrerer aktiver Substanzen ist.

12. Mittel nach Anspruch 11, dadurch gekennzeichnet, daß die wäßrigen Phasen D und E identisch sind.

13. Mittel nach einem der Ansprüche 11 oder 12, dadurch gekennzeichnet, daß die wäßrige Phase E oder die äußere wäßrige Phase D mindestens eine wasserlösliche kosmetische Substanz einschließen, ausgewählt unter Feuchthaltemitteln, künstlichen Bräunungsmitteln, Mitteln zur Kolorierung der Haut, Sonnenschutzmitteln, Sonnenfiltern, Antiperspirantien, desodorierenden Mitteln, astringierenden Mitteln, Erfrischungsprodukten, tonischen Produkten, Wundheilprodukten, keratolytischen Produkten, depilatorischen Produkten, Parfümwässern, wasserlöslichen Farbstoffen, Antischuppenmitteln, Antiseborrhömitteln, Oxidantien, Reduktionsmitteln und Extrakten aus tierischen oder pflanzlichen Geweben.

14. Mittel nach einem der Ansprüche 11 oder 12, dadurch gekennzeichnet, daß die wäßrige Phase E oder die äußere wäßrige Phase D mindestens ein Produkt einschließen, ausgewählt unter Vitaminen, Hormonen, Enzymen, Vaccinen, antiinflammatorischen Mitteln, Antibiotikas und Bakteriziden.

15. Mittel nach einem der Ansprüche 1 bis 14, dadurch gekennzeichnet, daß mindestens eine mit Wasser nicht mischbare Lipidphase L in der wäßrigen Phase D dispergiert ist.

16. Mittel nach Anspruch 15, dadurch gekennzeichnet, daß es 2 bis 70 Gew.-% der mit Wasser nicht mischbaren Lipidphase L, bezogen auf das Gesamtgewicht des Mittels, enthält, wobei das relative Gewichtsverhältnis des (der) die Kügelchen bildenden amphiphilen Lipids (Lipide), bezogen auf die mit Wasser nicht mischbare dispergierte Lipidphase, im Bereich von 0,02/1 bis 10/1 liegt.

17. Mittel nach einem der Ansprüche 15 oder 16, dadurch gekennzeichnet, daß die mit Wasser nicht mischbare, in der wäßrigen Phase D dispergierte Lipidphase L ausgewählt ist unter Ölen, wie Estern von Fettsäuren

mit Polyolen, und Estern von Fettsäuren und verzweigten Alkoholen der allgemeinen Formel $R_5$-$COOR_5$, worin $R_5$ den Rest einer höheren Fettsäure mit 7 bis 19 Kohlenstoffatomen bedeutet und $R_6$ eine verzweigte Kohlenwasserstoffkette bedeutet, die 3 bis 20 Kohlenstoffatome enthält, Kohlenwasserstoffen, wie Hexadekan, Paraffinöl, Perhydrosqualen ; halogenierten Kohlenstoffverbindungen, wie Perfluordecahydronaphthalin ; Perfluortributylamin ; Polysiloxanen ; Estern von organischen Säuren, Ethern und Polyethern.

18. Mittel nach einem der Ansprüche 1 bis 17, dadurch gekennzeichnet, daß die wäßrige Phase D mindestens ein Hilfsmittel, ausgewählt unter opakmachenden Mitteln, Gelbildnern, Aromastoffen, Parfüms, Sonnenfiltern und Farbstoffen, enthält.

19. Mittel nach einem der Ansprüche 15 bis 18, dadurch gekennzeichnet, daß die Phase L mindestens ein Parfüm und/oder eine oder mehrere fettlösliche aktive Substanzen enthält.

20. Mittel nach Anspruch 19, dadurch gekennzeichnet, daß die fettlösliche Substanz ein Sonnenfilter, eine Substanz zur Verbesserung trockener oder gealterter Haut, ausgewählt unter nicht-verseifbaren Sojaverbindungen, Advokatoverbindungen, Tocopherolen und den Vitaminen E und F oder einem Antioxidans ist.

## Claims

1. Cosmetic or pharmaceutical composition consisting of a dispersion in an aqueous medium D of lipid spherules consisting of organised molecular layers encapsulating an aqueous phase E, the lipid(s) constituting the said layers being ionic or nonionic amphiphiles, characterised in that the actual lipid phase of the said spherules is associated with at least one lipoprotide free from any mercapto functional group, chosen from the mono- or polyacylated derivatives of amino acids or of polypeptides, in which the acyl residue R-CO contains a hydrocarbon chain R which has 13 to 19 carbon atoms, at least one of the functional groups which joins the polypeptide chain or the amino acid residue to the lipophilic chain being an amide functional group, it being possible for the carboxylic functional groups of the polypeptide chain of the amino acid residue to be partially or completely neutralised by one or more alkali metal cations, an ammonium ion or a substituted ammonium derived from an amine, the said lipoprotide(s) being present in a proportion of 1 to 15% by weight relative to the total weight of the said actual lipid phase.

2. Composition according to Claim 1, characterised in that the acyl residue(s) of the lipoprotides employed is (or are) chosen from the myristoyl, palmitoyl, stearoyl, oleoyl, linoleoyl and linolenoyl residues.

3. Composition according to either of Claims 1 and 2, characterised in that the protide chain of the lipoprotides employed is derived from collagen or from hydroxyproline.

4. Composition according to Claim 1, characterised in that the lipoprotide(s) employed is (or are) chosen from the group made up of collagenic palmitoyl lipoaminoacid, the —O-N dipalmitoyl derivative of hydroxyproline, hydroxyproline linoleate, sodium stearoylglutamate, collagen stearoyltripeptide and collagen oleoyl-(tetra- or penta)peptide.

5. Composition according to one of Claims 1 to 4, characterised in that the lipids intended to constitute the leaflets of the spherules are ionic or nonionic amphiphiles of natural or synthetic origin, containing, per molecule, one or more hydrophilic group(s) taken from hydroxyl, ether oxide, carboxyl, phosphate and amine groups.

6. Composition according to Claim 5, characterised in that the ionic amphiphiles are taken from the group made up of natural phospholipids such as egg or soya lecithin and sphingomyelin, synthetic phospholipids, such as dipalmitoylphosphatidylcholine or hydrogenated lecithin, amphoteric compounds and anionic compounds.

7. Composition according to Claim 5, characterised in that the nonionic amphiphiles are taken from the group made up of :

— linear or branched polyglycerol ethers of respective formulae :

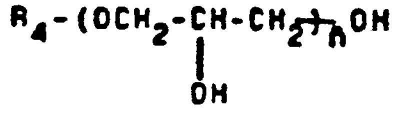

and

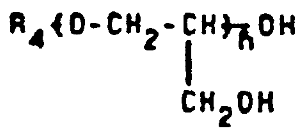

$\bar{n}$ being an average statistical value between 1 and 6, $R_4$ denoting a saturated or unsaturated, linear or branched, aliphatic chain containing 12 to 30 carbon atoms, hydrocarbon radicals of lanolin alcohols or the 2-hydroxyalkyl residues of long-chain α-diols containing from 8 to 30 carbon atoms ;

— linear or branched polyglycerol ethers containing two fatty chains ;
— polyoxyethylenated fatty alcohols or polyoxyethylenated sterols ;
— polyol ethers ;
— polyol esters, oxyethylenated or otherwise ;
— glycolipids of natural or synthetic origin ;
— the hydroxyamides denoted by the formula :

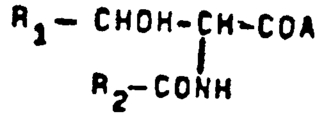

in which formula :
— $R_1$ denotes an alkyl or alkenyl radical containing 7 to 21 carbon atoms ;
— $R_2$ denotes a saturated or unsaturated hydrocarbon radical containing from 7 to 31 carbon atoms ;
— COA denotes a group chosen from the following two groups :
— a residue

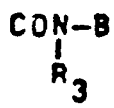

B being a radical derived from mono- or polyhydroxylated primary or secondary amines and $R_3$ denoting a hydrogen atom or a methyl, ethyl or hydroxyethyl radical ;
— COOZ, Z denoting the residue of a polyol containing from 3 to 7 carbon atoms.

8. Composition according to one of Claims 1 to 7, characterised in that the amphiphiles intended to form the spherules are associated with additives taken from the group made up of long-chain alcohols and diols containing from 8 to 30 carbon atoms, sterols, long-chain amines containing from 8 to 30 carbon atoms, hydroxyalkylamines, polyoxyethylenated fatty amines, esters of long-chain aminoalcohols containing from 8 to 30 carbon atoms, and their salts, fatty alcohol phosphoric esters, alkyl sulphates and ionic derivatives of sterols.

9. Composition according to one of Claims 1 to 8, characterised in that it contains from 0.5 to 25% by weight of amphiphile(s) constituting the spherule walls, these percentages being expressed by weight relative to the total weight of the composition.

10. Composition according to one of Claims 1 to 9, characterised in that the walls of these spherules contain at least one liposoluble substance such as, for example, keratolytic agents, antiinflammatory agents and antioxidant agents.

11. Composition according to one of Claims 1 to 10, characterised in that the aqueous phase E encapsulated in the spherules is an aqueous solution of active substance(s), preferably isoosmotic in relation to the phase D which surrounds the spherules.

12. Composition according to Claim 11, characterised in that the aqueous phases D and E are identical.

13. Composition according to either of Claims 11 and 12, characterised in that the aqueous phase E or the external aqueous phase D contains at least one watersoluble cosmetic substance taken from the group made up of humectants, artificial-tanning agents, skin-colouring agents, agents protecting against sunlight, sunscreens, antiperspirant agents, deodorants, astringents, refreshing products, tonic products, cicatrising products, keratolytic products, depilatory products, perfumed waters, water-soluble dyes, antidandruff agents, antiseborrhoeic agents, oxidising agents, reducing agents and animal or plant tissue extracts.

14. Composition according to either of Claims 11 and 12, characterised in that the aqueous phase E or

the external aqueous phase D contains at least one product taken from the group made up of vitamins, hormones, enzymes, vaccines, antiinflammatory agents, antibiotics and bactericides.

15. Composition according to one of Claims 1 to 14, characterised in that at least one liquid phase L, which is not miscible with water, is dispersed in the aqueous phase D.

16. Composition according to Claim 15, characterised in that it contains from 2 to 70% by weight of water-immiscible liquid phase L relative to the total weight of the composition, the relative weight proportion of amphiphilic lipid(s) constituting spherules being between 0.02/1 and 10/1 relative to the dispersed water-immiscible liquid phase.

17. Composition according to either of Claims 15 and 16, characterised in that the water-immiscible liquid phase L dispersed in the aqueous phase D is chosen from the group consisting of oils such as esters of fatty acids and of polyols and esters of fatty acids and of branched alcohols of formula $R_5$-$COOR_6$, in which formula $R_5$ denotes the residue of a higher fatty acid containing from 7 to 19 carbon atoms and $R_6$ denotes a branched hydrocarbon chain containing from 3 to 20 carbon atoms, hydrocarbons such as hexadecane, liquid paraffin, perhydrosqualene, halogenated hydrocarbons such as perfluorodecahydronaphthalene, perfluorotributylamine, polysiloxanes, organic acid esters, ethers and polyethers.

18. Composition according to one of Claims 1 to 17, characterised in that the aqueous phase D contains at least one adjuvant taken from the group made up of opacifiers, gelling agents, flavourings, perfumes, sunscreens and colourants.

19. Composition according to one of Claims 15 to 18, characterised in that the phase L contains at least one perfume and/or one or more liposoluble active substances.

20. Composition according to Claim 19, characterised in that the liposoluble substance consists of a sunscreen, a substance intended to improve the state of dry or senile skins, chosen from the unsaponifiables of soya, of avocado, tocopherols and vitamins E, F, or an antioxidant.